# EUROPEAN PATENT SPECIFICATION

(11) **EP 3 758 586 B1**
(45) Date of publication and mention of the grant of the patent: **28.07.2021**
(21) Application number: 19719670.2
(22) Date of filing: 27.03.2019
(51) Int. Cl.: A61B 5/00, A61B 5/0215, A61M 25/00

(54) **SENSOR GUIDE WIRE WITH THREE-HOLE JACKET FOR IMPROVED MANUFACTURABILITY AND REDUCED DRIFT**
SENSORFÜHRUNGSDRAHT MIT UMMANTELUNG MIT DREI ÖFFNUNGEN FÜR VERBESSERTE HERSTELLBARKEIT UND REDUZIERTEN DRIFT
FIL DE GUIDAGE DE CAPTEUR AVEC GAINE À TROIS TROUS POUR UNE FABRICABILITÉ AMÉLIORÉE ET UNE DÉRIVE RÉDUITE

(30) Priority: 17.04.2018 US 201862658677 P
(43) Date of publication of application: 06.01.2021
(73) Proprietor: St. Jude Medical Coordination Center BVBA, 1930 Zaventem (BE)
(72) Inventor: THOMAS, Ralph J., Champlin, Minnesota 55316 (US); MATEJKA, John A., Crystal, Minnesota 55414 (US); NESS, Peter J., Minneapolis, Minnesota 55417 (US)
(74) Representative: Zacco Sweden AB
(86) International application number: PCT/US2019/024292
(87) International publication number: WO 2019/203996

(56) References cited:
- WO-A1-2016/138226
- US-A1- 2013 102 927
- US-A1- 2013 296 718

## Description

### CROSS-REFERENCE TO RELATED APPLICATIONS

This application claims priority to and the benefit of US Provisional Application No. 62/658,677, filed April 17, 2018.

### BACKGROUND

The present disclosure relates to devices for making pressure or other measurements inside a living body, and in particular to a sensor/guide device having a guide wire and a distal sensor for pressure measurement in stenotic vessels.

Examples of such devices are described, for example, in Swedish Patents SE 441725, SE 453561, SE 454045, SE 460396, and SE 469454, in European Publication EP 0387453, in U.S. Patent No. 6,167,763, and in U.S. Patent Publication Nos. 2016/0249821, 2013/102927, and 2013/296718. US Patent Publication No. 2013/102927 discloses a sensor guide wire for intravascular measurement of a physiological variable comprising a sensor element arranged in a jacket, wherein the jacket comprises a number of openings.

U.S. Patent No. 6,167,763 describes a device in which a pressure sensor is mounted in a cantilevered configuration. In one embodiment, a tube extends around the pressure sensor. An opening is provided in the tube above the pressure sensor to allow the pressure sensor to be exposed to a surrounding medium, the pressure of which is to be measured.

U.S. Patent Publication No. 2016/0249821 explains that an unstable pressure column can form above the sensor transducer of the pressure sensor due to insufficient wetting when the device is immersed in a liquid. That publication hypothesized that instability in the pressure signal is caused by air pockets or bubbles trapped in or around the tube around the pressure sensor, and that this air influences the pressure column above the sensor transducer due to time dependent motion as a result of the inverse proportionality between capillary pressure and radius, as shown in the Young-Laplace equation, which describes the capillary pressure difference sustained across the interface between two static fluids due to surface tension. In other words, if the air pocket or bubble remained in place during the entire pressure measurement procedure, no problems would arise. But because the sensor guide wire is moved during the procedure, this can cause the air pocket or bubble to shift, which causes the pressure measurement to drift. To address this issue, U.S. Patent Publication No. 2016/0249821 describes various tube configurations that include multiple proximal openings and multiple distal openings extending through a circumferential wall of the tube surrounding the pressure sensor. These configurations provide certain benefits over prior tube designs.

However, the present inventors have discovered that there is a need for a further improved jacket design for sensor guide wires.

### SUMMARY

The present inventors have discovered that, while tube configurations that include multiple proximal openings and multiple distal openings may provide good drift characteristics, they can result in difficulties during assembly of the sensor guide wire. For example, as described in more detail below, during assembly of the sensor guide wire, the tube must be gripped while the tube is welded or otherwise attached to a core wire and/or an X-ray non-transparent distal coil. However, the tube is typically very small-about 2 mm in length and less than 1 mm in diameter. The present inventors have discovered that, if too many openings are included in the tube, the tube will be crushed as it is gripped during manufacturing. It was therefore necessary to determine a tube configuration that both prevents air pockets or bubbles from forming in the tube to reduce drift, but also allows for manufacturability. After substantial experimentation, the present inventors have discovered a tube design that results in an unexpectedly good combination of drift reduction and manufacturability.

In one embodiment, a guide wire for biological pressure measurement includes: a tube extending along an axial direction of the guide wire, wherein the tube comprises a circumferential wall and defines a proximal-end opening and a distal-end opening; and a pressure sensor configured for biological pressure measurement, wherein at least a portion of the pressure sensor is mounted within the tube, and wherein the pressure sensor comprises a pressure sensor transducer, wherein the circumferential wall of the tube includes exactly three openings configured for fluid and gas ingress and egress extending radially therethrough: a first elongated opening located at a location opposing the pressure sensor transducer such that the pressure sensor transducer is exposed via the first elongated opening, a second elongated opening located such that, in a cross-sectional view, a bisector of the second elongated opening is offset from a bisector of the first elongated opening in a first circumferential direction by an angle in a range of 95 to 115 degrees, and a third elongated opening located such that, in the cross-sectional view, a bisector of the third elongated opening is offset from the bisector of the first elongated opening in a second circumferential direction by an angle in a range of 95 to 115 degrees. Each of the first, second, and third elongated openings is elongated in the axial direction of the guide wire, wherein a majority of each of the first, second, and third elongated openings is located within a distal half of the tube. In the cross-sectional view, a circumferential angle over which the first elongated opening extends is greater than a circumferential angle over which the second elongated opening extends and a circumferential angle over which the third elongated opening extends.

In one aspect, in the cross-sectional view, the circumferential angle over which the first elongated opening extends is in a range of 65 to 75 degrees.

In one aspect, with is combinable with any combination of the above embodiments and aspects, in the cross-sectional view, the circumferential angle over which the second elongated opening extends and the circumferential angle over which the third elongated opening extends are each in a range of 35 to 45 degrees.

In one aspect, with is combinable with any combination of the above embodiments and aspects, in the cross-sectional view, a length of a chord extending between opposing edges of the first elongated opening is in a range of 0.18 to 0.22 mm.

In one aspect, with is combinable with any combination of the above embodiments and aspects, in the cross-sectional view, a length of a chord extending between opposing edges of the second elongated opening and a length of a chord extending between opposing edges of the third elongated opening are each in a range of 0.07 to 0.17 mm.

In one aspect, with is combinable with any combination of the above embodiments and aspects, an outer diameter of the tube is in a range of 0.33 to 0.39 mm.

In one aspect, with is combinable with any combination of the above embodiments and aspects, an inner diameter of the tube is in a range of 0.26 to 0.32 mm.

In one aspect, with is combinable with any combination of the above embodiments and aspects, a thickness of the circumferential wall is in a range of 0.030 to 0.040 mm.

In one aspect, with is combinable with any combination of the above embodiments and aspects, portions of the tube form a first strut located between the first elongated opening and the second elongated opening, and a second strut located between the first elongated opening and the third elongated opening, and in the cross-sectional view, the circumferential angle over which the first strut extends and the circumferential angle over which the second strut extends are each in a range of 40 to 50 degrees.

In one aspect, with is combinable with any combination of the above embodiments and aspects, in the cross-sectional view, a length of a chord extending between opposing edges of the first strut and a length of a chord extending between opposing edges of the second strut are each in a range of 0.08 to 0.22 mm.

In one aspect, with is combinable with any combination of the above embodiments and aspects, proximal ends of the first, second, and third elongated openings are aligned in a circumferential direction, and distal ends of the first, second, and third elongated openings are aligned in a circumferential direction.

In one aspect, with is combinable with any combination of the above embodiments and aspects, an axial distance between a distal end of the tube and each of the distal ends of the first, second, and third elongated openings is in a range of 0.18 to 0.28 mm.

In one aspect, with is combinable with any combination of the above embodiments and aspects, an axial distance between the distal end of the tube and each of the proximal ends of the first, second, and third elongated openings is in a range of 0.84 to 0.94 mm.

In one aspect, with is combinable with any combination of the above embodiments and aspects, an axial length of the tube is in a range of 1.8 to 2.2 mm.

In one aspect, with is combinable with any combination of the above embodiments and aspects, an axial length of the first elongated opening, an axial length of the second elongated opening, and an axial length of the third elongated opening are each in a range of 27 to 37% of a length of the tube.

In one aspect, with is combinable with any combination of the above embodiments and aspects, an axial length of the first elongated opening, an axial length of the second elongated opening, and an axial length of the third elongated opening are each in a range of 0.55 to 0.75 mm.

In one aspect, with is combinable with any combination of the above embodiments and aspects, in a top view, an axial distance between a proximal end of the pressure sensor transducer and a proximal end of the first elongated opening is in a range of 0.23 to 0.53 mm.

In one aspect, with is combinable with any combination of the above embodiments and aspects, wherein the first elongated opening has a shape of a rectangle with rounded corners.

In one aspect, with is combinable with any combination of the above embodiments and aspects, each of the second and third elongated openings has a shape of a rectangle with circular segments at proximal and distal ends thereof.

In one aspect, with is combinable with any combination of the above embodiments and aspects, a radius of the rounded corners and a radius of the circular segments are each in a range of 0.04 to 0.06 mm.

In one aspect, with is combinable with any combination of the above embodiments and aspects, in the cross-sectional view, the bisector of the second elongated opening is offset from the bisector of the first elongated opening in the first circumferential direction by an angle in a range of 95 to 105 degrees, and in the cross-sectional view, the bisector of the third elongated opening is offset from the bisector of the first elongated opening in the second circumferential direction by an angle in a range of 95 to 105 degrees.

In one aspect, with is combinable with any combination of the above embodiments and aspects, an entirety of each of the first, second, and third elongated openings is located within the distal half of the tube.

### BRIEF DESCRIPTION OF THE DRAWINGS

FIG. 1 is a side cross-sectional view of a prior art guide wire including a tube and pressure sensor.
FIG. 2 a side cross-sectional view of a sensor portion of a prior art guide wire similar to that shown in FIG. 1.
FIGS. 3A and 3B are side perspective views of a portion of a guide wire that includes a tube having exactly three openings configured for fluid and gas ingress and egress extending through a circumferential wall of the tube, according to an exemplary embodiment, FIG. 3A showing the tube alone, and FIG. 3B showing a portion of a guide wire that includes the tube, a pressure sensor, a core wire, and a distal coil.
FIG. 4A is a top view of the tube shown in FIGS. 3A and 3B, and FIG. 4B is a top view of the tube along with a distal coil, a core wire, a sensor element with a sensor transducer, and a proximal tubing portion.
FIG. 5 is a side cross-sectional view of the tube shown in FIGS. 3A and 3B, taken along the line B-B in FIG. 4A.
FIGS. 6A and 6B are front cross-sectional views of the tube shown in FIGS. 3A and 3B, taken along the line A-A in FIG. 4A.
FIGS. 7A-7D are graphs of drift (mm-Hg) over time (min) resulting from experimental testing performed on four different tube designs, FIG. 7A showing results of testing performed on a one-opening tube design, FIG. 7B showing results of testing performed on a two-opening tube design, FIG. 7C showing results of testing performed on a three-opening tube design according to an embodiment of the invention, and FIG. 7D showing results of testing performed on a four-opening tube design.

### DETAILED DESCRIPTION

Referring generally to the figures, a guide wire used for biological pressure measurements is shown and described.

### General Configuration of Pressure Sensor Guide Wires

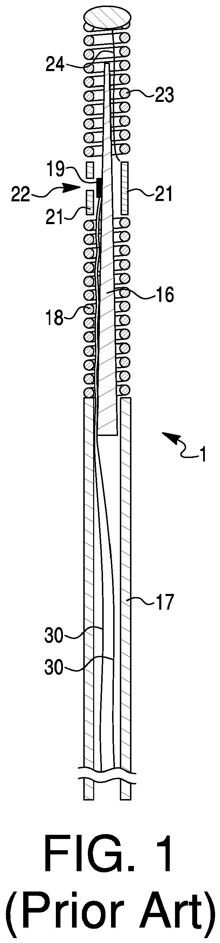

Referring to FIG. 1, a cross section of a prior art guide wire 1 is shown. The guide wire 1 includes a solid core wire 16 disposed in a portion of a proximal tubing portion 17. The core wire 16 may, for example, be machined by centering grinding. The solid core wire 16 may form the distal portion of the guide wire 1 and extend beyond the distal end of the proximal tubing portion 17. The proximal tubing portion 17 may be connected to or integrally formed with an optional proximal coil 18.

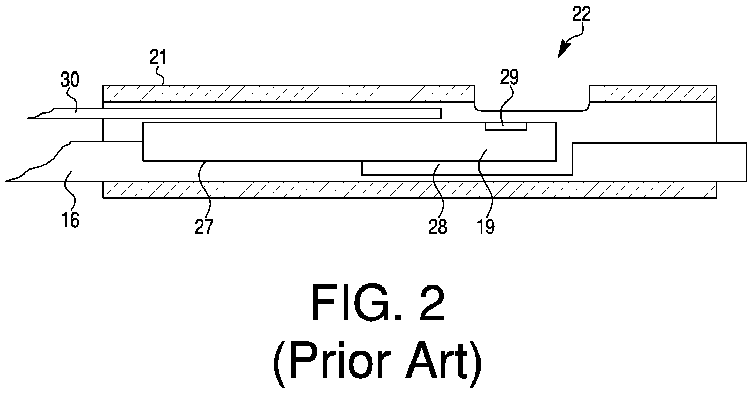

A pressure sensor element 19 is mounted on the wire 16. The pressure sensor element 19 may be an absolute pressure sensor, such as the one disclosed in Swedish patent application No. 9600334-8. The pressure sensor element 19 may include a transducer 29 such as the membrane shown in FIG. 2, which may be made of, for example, polysilicon and a piezoresistive element. Between the wire 16 and the proximal coil 18, one or more leads 30 may run from the electronic circuitry of the pressure sensor element 19. The wire 16, may act as a portion of one of the leads 30, as in FIG. 1.

The transducer of the pressure sensor element 19 may be mounted such that bending artifacts are minimized or eliminated (e.g., ensuring that the edges of the pressure sensor element 19 do not come into contact with the surrounding tube).

The pressure sensor element 19 is protected by a short section of a tube 21, having an aperture 22 through which a surrounding medium interacts with the pressure sensor element 19. The guide wire 1 further includes, at the very distal end of the guide wire, an X-ray non-transparent distal coil 23 (also referred to as a "radiopaque coil"), made, for example, of platinum, and used for location purposes, and a safety wire 24 for securing the distal part of the coil 23.

In one embodiment, the wire 16 is made of stainless steel. In other embodiments, the wire 16 may be made of a shape memory metal. The proximal tubing 17 and the proximal coil 18 may be coupled so as to be utilized as an electrical shield.

Referring to FIG. 2, a prior art sensor arrangement of the guide wire 1 is illustrated. The core wire 16 may be machined (e.g., by grinding, spark erosion, or laser techniques) to form a groove in which the sensor element is mounted in a cantilevering fashion. The groove provides a free space for the sensor element 19 and transducer 29 (in this case a membrane or diaphram), allowing air, blood, or other pressure exerting mediums to enter the interior of the guide wire and act on the sensor, which in turn delivers a signal representative of the exerted pressure.

The groove may have two portions. A first portion of the groove may serve as a shelf 27 for receiving the proximal part of the sensor chip and holding the sensor in place. The second portion 28 may be an open space over which the cantilever extends, which allows the distal part of the sensor chip to protrude freely, even in a case where the wire tip is bent or deflected.

As shown in FIGS. 1 and 2, a tube 21 is provided around the guide wire 1, the tube including an opening 22 used to expose the sensor chip to a surrounding medium (e.g., fluid) in order to measure the pressure of the medium.

The prior art embodiments of FIGS. 1 and 2 have a single aperture or opening 22 in the guide wire through which the pressure sensor element 19 is exposed to the surrounding medium. However, as discussed above, the presence of air in and around the guide wire 1 may cause unstable sensor readings.

### Guide Wires According to Embodiments of the Invention

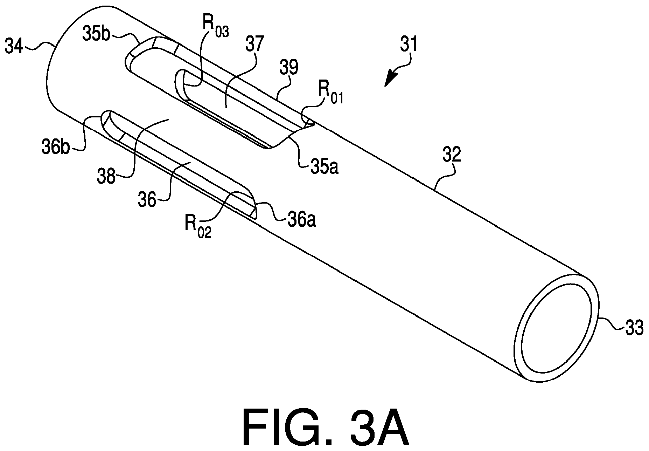

Referring to FIGS. 3A-6B, embodiments of a guide wire 300 according to embodiments of the present invention will be described. The guide wire generally includes a tube 31 with exactly three openings 35, 36, 37 configured for fluid and gas ingress and egress extending through a circumferential wall 32. As will be described in detail below, the tube 31 provides a surprisingly good combination of drift reduction and manufacturability.

In the embodiments described below, the guide wire 300 may further include a pressure sensor element 19, a proximal tubing portion 17, a proximal coil 18, an X-ray non-transparent distal coil 23, leads 30, etc., as generally described with respect to FIGS. 1 and 2 and discussed in U.S. Patent No. 6,167,763. In other words, the guide wire 300 according to embodiments of the invention may be identical to that shown in FIGS. 1 and 2, except for the tube 21 in FIGS. 1 and 2 being replaced by the tube 31 described below with reference to FIGS. 3A-6B. While the pressure sensor element 19 described above is a piezoelectric transducer type sensor (in this case, a piezoelectric membrane type sensor), the tube 31 described herein is also applicable to a variety of other sensor types. For example, the pressure sensor may be a fiber optic-based pressure sensor or any other sensor suitable for a guide wire or microcatheter.

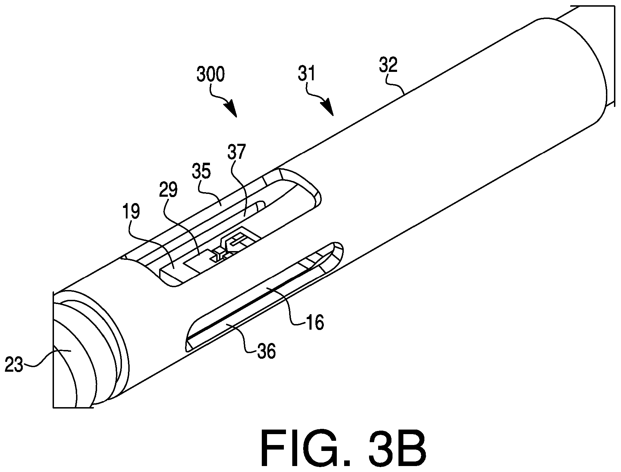

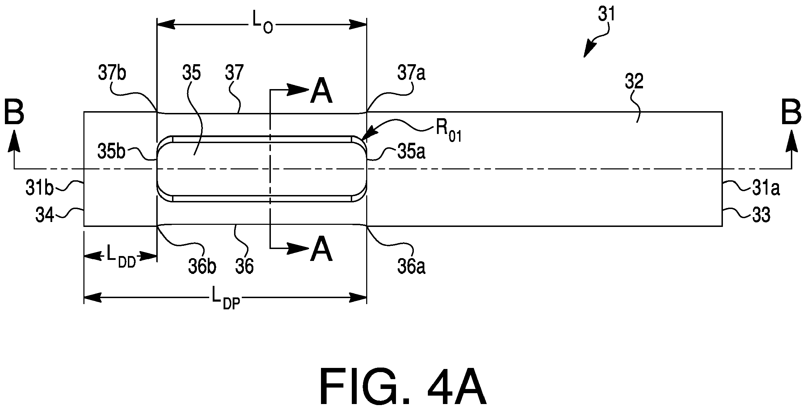

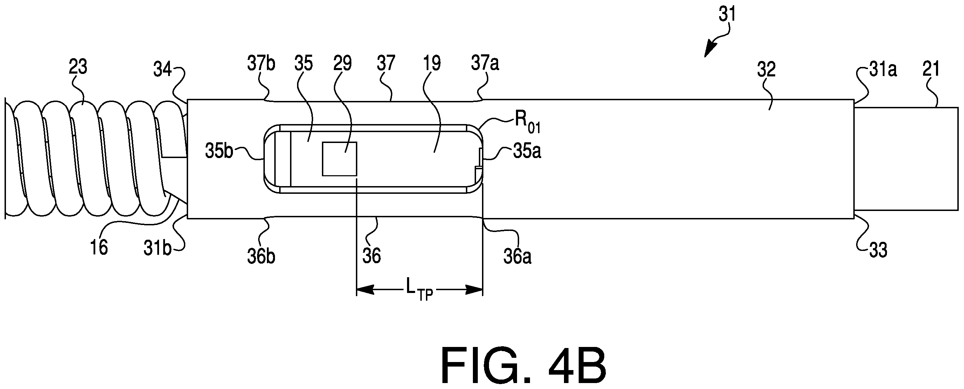

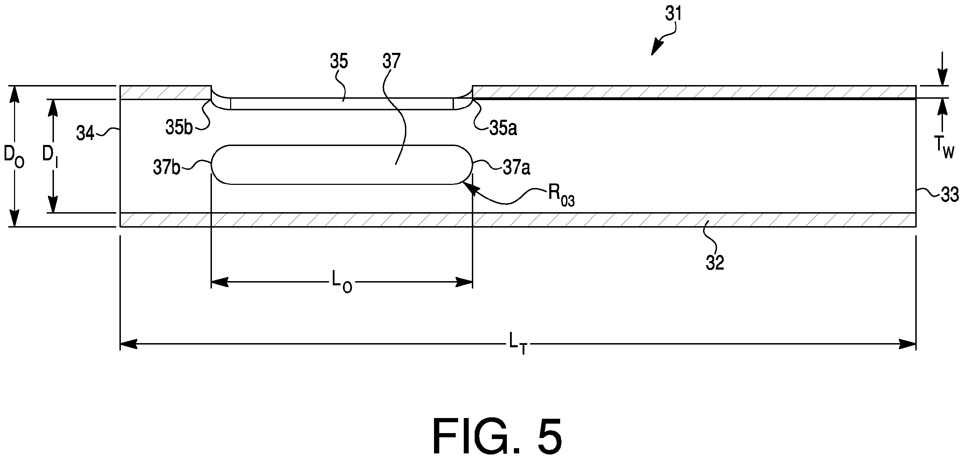

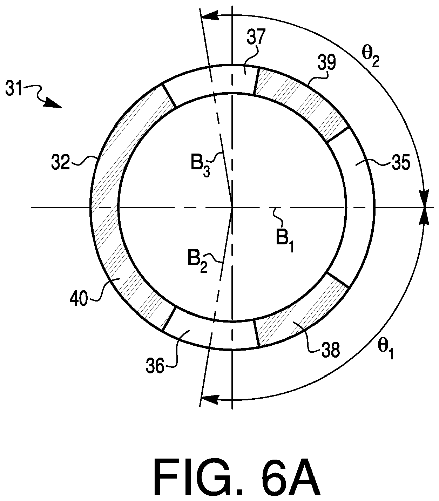

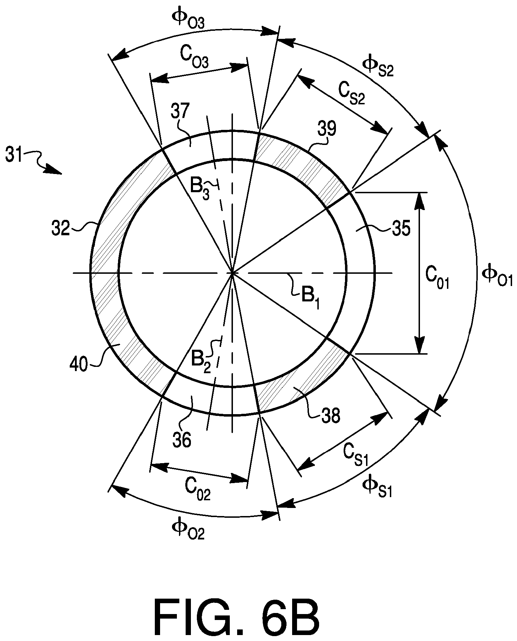

FIGS. 3A and 3B are side perspective views of a portion of a guide wire 300 that includes a tube having exactly three openings configured for fluid and gas ingress/egress extending through a circumferential wall of the tube, according to an exemplary embodiment, FIG. 3A showing the tube alone, and FIG. 3B showing a portion of a guide wire 300 that includes the tube, a pressure sensor, a core wire, and a distal coil. FIG. 4A is a top view of the tube shown in Figure 3. FIG. 4B is a top view of the tube along with a distal coil, a core wire, a sensor element with a sensor transducer, and a proximal tubing portion. FIG. 5 is a side cross-sectional view of the tube shown in FIGS. 3A and 3B, taken along the line B-B in FIG. 4A. FIGS. 6A and 6B are front cross-sectional views of the tube shown in FIGS. 3A and 3B, taken along the line A-A in FIG. 4A.

According to one embodiment, a guide wire 300 for biological pressure measurement includes a tube 31 extending along an axial direction of the guide wire 300, as shown in all of FIGS. 3A-6B. The tube 31 comprises a circumferential wall 32 and defines a proximal-end opening 33 and a distal-end opening 34. The guide wire 300 further includes a pressure sensor element 19 configured for biological pressure measurement, as shown in FIG. 3B. At least a portion of the pressure sensor element 19 is mounted within the tube 31. The pressure sensor element 19 includes a pressure sensor transducer 29.

The circumferential wall of the tube includes exactly three openings configured for fluid and gas ingress and egress extending radially therethrough: a first elongated opening 35, a second elongated opening 36, and a third elongated opening 37. The first elongated opening 35 is located at a position opposing the pressure sensor transducer 29 such that the pressure sensor transducer 29 is exposed via the first elongated opening 35. As shown in FIG. 6A, the second elongated opening 36 is located such that, in a cross-sectional view (as shown, for example, in FIGS. 6A and 6B), a bisector B₂ of the second elongated opening 36 is offset from a bisector B₁ of the first elongated opening 35 in a first circumferential direction (clockwise in this example) by an angle θ₁ in a range of 95 to 115 degrees, and preferably 95 to 105 degrees. As also shown in FIG. 6A, the third elongated opening 37 is located such that, in the cross-sectional view, a bisector B₃ of the third elongated opening 37 is offset from the bisector B₁ of the first elongated opening 35 in a second circumferential direction (counterclockwise in this example) by an angle θ₂ in a range of 95 to 115 degrees, and preferably 95 to 105 degrees. As shown in FIGS. 6A and 6B, a first strut 38 is located between the first elongated opening 35 and the second elongated opening 36, a second strut 39 is located between the first elongated opening 35 and the third elongated opening 37, and a third strut 40 is located between the second elongated opening 36 and the third elongated opening 37. By setting the angles θ₁ and θ₂ at 95 degrees or greater, it is possible to allow the circumferential angles over which the struts 38, 39, 40 extend to be large enough to provide sufficient strength to the tube 31, but still allow the circumferential angles over which the openings 35, 36, 37 extend to be sufficiently large to allow air to escape from the tube 31 and provide sufficient wetting of the sensor transducer 29.

Each of the first, second, and third elongated openings 35, 36, 37 is elongated in the axial direction of the guide wire 300. By making the openings 35, 36, 37 elongated, air can more easily escape the tube 31 via the openings 35, 36, 37.

A majority of each of the first, second, and third elongated openings 35, 36, 37 is located within a distal half of the tube 31. Because a majority of the openings 35, 36, and 37 are located within a distal half of the tube 31, the strength of the tube 31 is increased over those in which the openings are also included in the proximal half of the tube. Preferably an entirety of each of the first, second, and third elongated openings 35, 36, 37 is located within a distal half of the tube 31, which can provide even more strength to the tube.

As shown in Figures 6A and 6B, in the cross-sectional view, a circumferential angle ϕ_{O1} over which the first elongated opening 35 extends is greater than a circumferential angle ϕ_{O2} over which the second elongated opening 36 extends and a circumferential angle ϕ_{O3} over which the third elongated opening 37 extends.

In the cross-sectional view, the circumferential angle ϕ_{O1} over which the first elongated opening 35 extends may be in a range of 60 to 80 degrees, and preferably in a range of 65 to 75 degrees.

In the cross-sectional view, the circumferential angle ϕ_{O2} over which the second elongated opening 36 extends and the circumferential angle ϕ_{O3} over which the third elongated opening 37 extends may be each in a range of 30-50 degrees, and preferably in a range of 35 to 45 degrees.

In the cross-sectional view, a length of a chord Coi extending between opposing edges of the first elongated opening 35 may be in a range of 0.16 to 0.24 mm, and preferably in a range of 0.18 to 0.22 mm.

In the cross-sectional view, a length of a chord C_{O2} extending between opposing edges of the second elongated opening 36 and a length of a chord C_{O3} extending between opposing edges of the third elongated opening 37 may be each in a range of 0.05 to 0.19 mm, and preferably in a range of 0.07 to 0.17 mm.

An axial length of the tube may be in a range of 1.8 to 2.2 mm. An outer diameter D_{O} of the tube 31 may be in a range of 0.30 to 0.42 mm, and preferably in a range of 0.33 to 0.39 mm. An inner diameter D_{I} of the tube 31 may be in a range of 0.23 to 0.35 mm, and preferably in a range of 0.26 to 0.32 mm.

A thickness T_{W} of the circumferential wall 32 may be in a range of 0.017 to 0.047 mm.

In the cross-sectional view, the circumferential angle ϕ_{S1} over which the first strut 38 extends and the circumferential angle ϕ_{S2} over which the second strut 39 extends may be each in a range of 35 to 55 degrees, and preferably in a range of 40 to 50 degrees.

In the cross-sectional view, a length of a chord C_{S1} extending between opposing edges of the first strut 38 and a length of a chord C_{S2} extending between opposing edges of the second strut 39 may be each in a range of 0.08 to 0.22 mm, and preferably in a range of 0.12 to 0.16 mm.

Proximal ends 35a, 36a, 37a of the first, second, and third elongated openings 35, 36, 37 are aligned in a circumferential direction, and distal ends 35b, 36b, 37b of the first, second, and third elongated openings 35, 36, 37 are aligned in a circumferential direction.

The tube 31 has a proximal end 31a and a distal end 31b, as shown in FIGS. 4A and 4B. An axial distance L_{DD} between a distal end 31b of the tube 31 and each of the distal ends 35a, 35b, 35c of the first, second, and third elongated openings 35, 36, 37 may be in a range of 0.13 to 0.33 mm, and preferably in a range of 0.18 to 0.28 mm. An axial distance L_{DP} between the distal end 31b of the tube 31 and each of the proximal ends 35a, 36a, 37a of the first, second, and third elongated openings 35, 36, 37 may be in a range of 0.79 to 0.99 mm, and preferably in a range of 0.84 to 0.94 mm.

Axial lengths L_{O} of the fi first, second, and third elongated openings 35, 36, 37 may be each in a range of 22% to 42 % of a length L_{T} of the tube 31, and preferably in a range of 27 to 37% of a length L_{T} of the tube 31. Axial lengths Lo of the first, second, and third elongated openings 35, 36, 37 may be each in a range of 0.55 to 0.75 mm.

As shown in FIG. 4B, in a top view, an axial distance L_{TP} between a proximal end of the pressure sensor transducer and a proximal end 35a of the first elongated opening 35 may be in a range of 0.23 to 0.53 mm, and preferably in a range of 0.28 to 0.48 mm.

The first elongated opening 35 may have a shape of a rectangle with rounded corners. Each of the second and third elongated openings 36, 37 may have a shape of a rectangle with circular segments at proximal and distal ends 36a, 36b, 37a, 37b thereof.

A radius R_{O1} of the rounded corners of the first elongated opening 35 and a radius R_{O2}, R_{O3} of the circular segments may be each in a range of 0.04 to 0.06 mm.

### Results of Experimental Testing

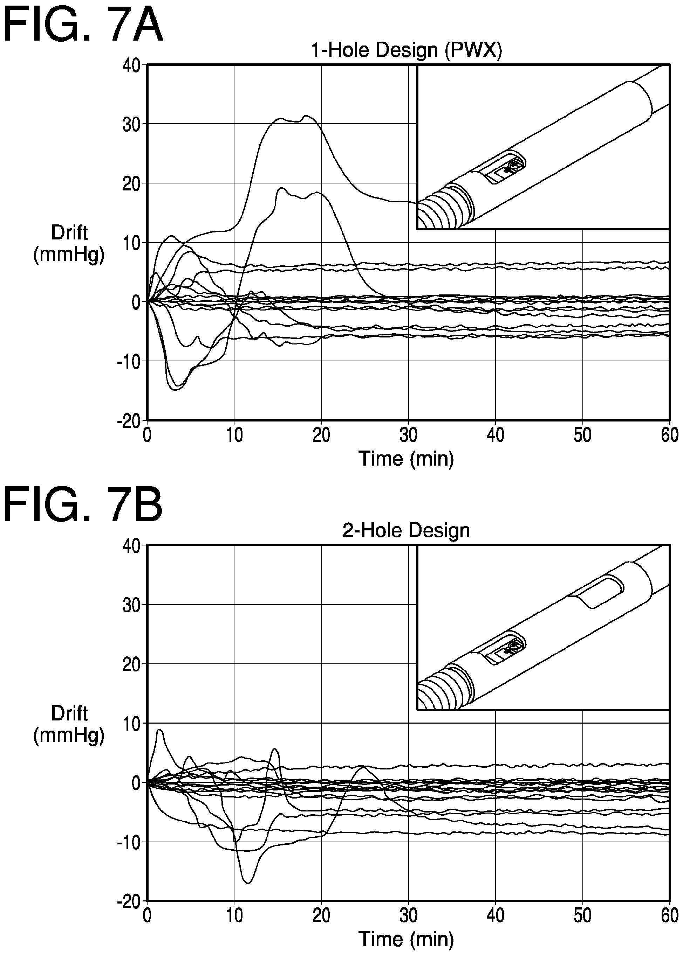

The tube 31 provides a surprisingly good combination of drift reduction and manufacturability when included in a sensor guide wire 300. These unexpected results are demonstrated by experimental testing performed on sensor guide wires with tubes having various hole configurations. The tested tubes included (i) a first tube having a single opening extending through a circumferential wall of the tube, (ii) a second tube having two openings extending through the circumferential wall of the tube, (iii) a third tube having three openings extending through the circumferential wall of the tube (according to an embodiment of the present invention), and (iv) a fourth tube having four openings extending through the circumferential wall of the tube. The specific designs of the tested tubes are shown in the insets in FIGS. 7A-7D.

The first tube was a one-opening tube having exactly one opening extending through the circumferential wall of the tube, as in the prior art guide wire shown in FIGS. 1 and 2.

The second tube was a two-opening tube having a distal opening an a top distal side of the tube, to be located opposing the sensor membrane of a pressure sensor element, and a proximal opening on a top proximal side of the tube, to be located opposing a silicone coating on the pressure sensor element.

The third tube was a tube 31 having exactly three openings as described above with respect to embodiments of the present invention.

The fourth tube was a four-opening tube having a distal opening an a top distal side of the tube, to be located opposing the sensor membrane of a pressure sensor element, a proximal opening on a top proximal side of the tube, to be located opposing a silicone coating on the pressure sensor element, and two side openings located near a middle of the tube.

First, manufacturability of sensor guide wires using the two-opening, three-opening, and four-opening tubes was tested.

Substantial manufacturing difficulties arose during manufacturing with the two-opening tube. Specifically, the separation of the windows between the proximal and distal ends of the tube made it difficult to clean joints during a joint cleaning process after the tube was bonded to other portions of the guide wire.

Further, in both the two-opening tube and the four-opening tube, it was difficult to remove adhesive particles left on the silicone coating that was exposed via the proximal opening. And in all tubes with proximal openings, including the two-opening tube and the four opening tube, it was difficult to clamp the tube during assembly, because bending occurred at much lower clamping forces.

Second, sensor guide wires using all four of the tube designs were tested for drift performance. For each tested guide wire, 25 tests were performed. The test method and apparatus was designed to simulate human blood pressure and flow rates. The pressure for systolic and diastolic pressure were set for test purposes at 120/80 mmHg. Pressure was not varies once the test was started. The devices were positioned at the same spot for all devices and left there for the duration of the test. In each test, pressure was measured continuously for 60 minutes. Drift reduces the accuracy of the intravascular pressure measurement. Reduction of drift improves accuracy, and thus improves a doctor's ability to evaluate the stenosis and determine the best course of treatment for a patient.

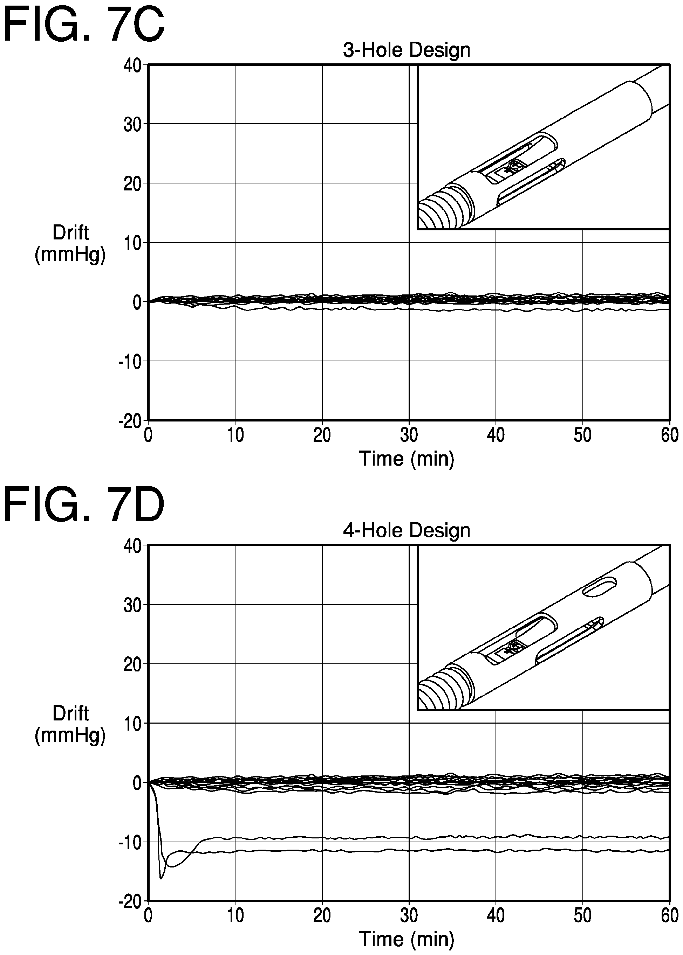

The results of the drift testing are shown in FIGS. 7A-7D, which are graphs of drift (mm-Hg) over time (min.). FIG. 7A shows results of testing performed on the one-opening tube design, FIG. 7B shows results of testing performed on the two-opening tube design, FIG. 7C shows results of testing performed on the three-opening tube design according to an embodiment of the invention, and FIG. 7D shows results of testing performed on the four-opening tube design. The results of the testing are compiled in Table 1 below.

**Table 1:Drift Mean, Drift Standard Deviation (StDev), and Number of Bubbles**

| **Design** | **Mean** | **StDev** | **Bubbles** |
|---|---|---|---|
| **4-hole** | 1.87 | 3.72 | 3 |
| **3-hole** | 0.77 | 0.32 | 0 |
| **2-hole** | 4.01 | 5.34 | 14 |
| **1-hole** | 6.40 | 9.31 | 15 |

The guide wires with one-opening, two-opening, and four-opening tubes all experienced substantial pressure drift during the testing, with a mean pressure drift of 6.40 mmHg, 4.01 mmHg, and 1.87 mmHg, respectively. The three-opening tube design yielded a mean pressure drift of only 0.77- an 81% improvement over the two-hole design. This alone is a surprising improvement. But even more surprising was the fact that the three-opening design yielded a ***59% reduction in drift*** relative to the four-opening design.

As demonstrated above, the three-opening design described in the present application yields a surprisingly good combination of manufacturability and pressure drift reduction. The present inventors believe that these results are an effect of the specific configuration of the tube 31 in which the first elongated opening 35 is located at a location opposing the pressure sensor membrane 29 such that the pressure sensor membrane 29 is exposed via the first elongated opening 35, the second elongated opening 36 is located such that, in a cross-sectional view, a bisector B₂ of the second elongated opening 36 is offset from a bisector B₁ of the first elongated opening 35 in a first circumferential direction by an angle θ₁ in a range of 95 to 115 degrees, the third elongated opening 37 is located such that, in the cross-sectional view, a bisector B₃ of the third elongated opening 37 is offset from the bisector B₁ of the first elongated opening 35 in a second circumferential direction by an angle θ₂ in a range of 95 to 115 degrees, each of the first, second, and third elongated openings 35, 36, 37 is elongated in the axial direction of the guide wire 300, a majority of each of the first, second, and third elongated openings 35, 36, 37 is located within a distal half of the tube 31, and in the cross-sectional view, a circumferential angle ϕ₁ over which the first elongated opening 35 extends is greater than a circumferential angle ϕ₂ over which the second elongated opening 36 extends and a circumferential angle ϕ₃ over which the third elongated opening 37 extends.

## Claims

1. A guide wire (300) for biological pressure measurement comprising:
a tube (31) extending along an axial direction of the guide wire (300), wherein the tube (31) comprises a circumferential wall (32) and defines a proximal-end opening (33) and a distal-end opening (34); and
a pressure sensor (19) configured for biological pressure measurement, wherein at least a portion of the pressure sensor (19) is mounted within the tube (31), and wherein the pressure sensor (19) comprises a pressure sensor transducer (29),
the circumferential wall (32) of the tube (31) includes exactly three openings (35, 36, 37) configured for fluid and gas ingress and egress extending radially therethrough: a first elongated opening (35), a second elongated opening (36), and a third elongated openings (37),
wherein each of the first, second, and third elongated openings (35, 36, 37) is elongated in the axial direction of the guide wire (300),
wherein a majority of each of the first, second, and third elongated openings (35, 36, 37) is located within a distal half of the tube (31),
the first elongated opening (35) is located at a location opposing the pressure sensor transducer (29) such that the pressure sensor transducer (29) is exposed via the first elongated opening (35),
**characterized in that**
the second elongated opening (36) is located such that, in a cross-sectional view, a bisector (B₂) of the second elongated opening (36) is offset from a bisector (B₁) of the first elongated opening (35) in a first circumferential direction by an angle (θ₁) in a range of 95 to 115 degrees, and
the third elongated opening (37) is located such that, in the cross-sectional view, a bisector (B₃) of the third elongated opening (37) is offset from the bisector (B₁) of the first elongated opening (35) in a second circumferential direction by an angle (θ₂) in a range of 95 to 115 degrees,
wherein, in the cross-sectional view, a circumferential angle (ϕ_{O1}) over which the first elongated opening (35) extends is greater than a circumferential angle (ϕ_{O2}) over which the second elongated opening (36) extends and a circumferential angle (ϕ_{O3}) over which the third elongated opening (37) extends.

2. The guide wire (300) of claim 1, wherein, in the cross-sectional view, the circumferential angle (ϕ_{O1}) over which the first elongated opening (35) extends is in a range of 65 to 75 degrees, and the circumferential angle (ϕ_{O2}) over which the second elongated opening (36) extends and the circumferential angle (ϕ_{O3}) over which the third elongated opening (37) extends are each in a range of 35 to 45 degrees.

3. The guide wire (300) of claim 1 or claim 2, wherein, in the cross-sectional view, a length of a chord (C_{O1}) extending between opposing edges of the first elongated opening (35) is in a range of 0.18 to 0.22 mm, and a length of a chord (C_{O2}) extending between opposing edges of the second elongated opening (36) and a length of a chord (C_{O3}) extending between opposing edges of the third elongated opening (37) are each in a range of 0.07 to 0.17 mm.

4. The guide wire (300) of any of claims 1 -3, wherein an outer diameter of the tube (31) is in a range of 0.33 to 0.39 mm, and an inner diameter of the tube (31) is in a range of 0.26 to 0.32 mm.

5. The guide wire (300) of any of claims 1-4, wherein:
portions of the tube (31) form a first strut (38) located between the first elongated opening (35) and the second elongated opening (36), and a second strut (39) located between the first elongated opening (35) and the third elongated opening (37), and
in the cross-sectional view, the circumferential angle (ϕ_{S1}) over which the first strut (38) extends and the circumferential angle (ϕ_{S2}) over which the second strut (39) extends are each in a range of 40 to 50 degrees.

6. The guide wire (300) of claim 5, wherein, in the cross-sectional view, a length of a chord (C_{S1}) extending between opposing edges of the first strut (38) and a length of a chord (C_{S2}) extending between opposing edges of the second strut (39) are each in a range of 0.08 to 0.22 mm.

7. The guide wire (300) of any of claims 1-6, wherein proximal ends (35a, 36a, 37a) of the first, second, and third elongated openings (35, 36, 37) are aligned in a circumferential direction, and distal ends (35b, 36b, 37b) of the first, second, and third elongated openings (35, 36, 37) are aligned in a circumferential direction.

8. The guide wire (300) of claim 7, wherein an axial distance (L_{DD}) between a distal end (31b) of the tube (31) and each of the distal ends (35b, 36b, 37b) of the first, second, and third elongated openings (35, 36, 37) is in a range of 0.18 to 0.28 mm.

9. The guide wire (300) of claim 8, wherein an axial distance (L_{DP}) between the distal end (31b) of the tube (31) and each of the proximal ends (35a, 36a, 37a) of the first, second, and third elongated openings (35, 36, 37) is in a range of 0.84 to 0.94 mm.

10. The guide wire (300) of any of claims 1-9, wherein an axial length (L_{O}) of the first elongated opening (35), an axial length (L_{O}) of the second elongated opening (36), and an axial length (L_{O}) of the third elongated opening (37) are each in a range of 27 to 37% of a length of the tube (31).

11. The guide wire (300) of any of claims 1-10, wherein an axial length (L_{O}) of the first elongated opening (35), an axial length (L_{O}) of the second elongated opening (36), and an axial length (Lo) of the third elongated opening (37) are each in a range of 0.55 to 0.75 mm.

12. The guide wire (300) of any of claims 1-11, wherein the first elongated opening (35) has a shape of a rectangle with rounded corners.

13. The guide wire (300) of claim 12, wherein each of the second and third elongated openings (35, 36, 37) has a shape of a rectangle with circular segments at proximal and distal ends thereof.

14. The guide wire (300) of any of claims 1-13, wherein:
in the cross-sectional view, the bisector (B₂) of the second elongated opening (36) is offset from the bisector (B₁) of the first elongated opening (35) in the first circumferential direction by an angle (θ₁) in a range of 95 to 105 degrees, and
in the cross-sectional view, the bisector (B₃) of the third elongated opening (37) is offset from the bisector (B₁) of the first elongated opening (35) in the second circumferential direction by an angle (θ₂) in a range of 95 to 105 degrees.

15. The guide wire (300) of any of claims 1-14, wherein an entirety of each of the first, second, and third elongated openings (35, 36, 37) is located within the distal half of the tube (31).

## Patentansprüche

1. Führungsdraht (300) zur Messung biologischen Drucks umfassend:
ein sich entlang einer Axialrichtung des Führungsdrahts (300) erstreckendes Rohr (31), wobei das Rohr (31) eine Umfangswandung (32) umfasst und eine Öffnung (33) am proximalen Ende und eine Öffnung (34) am distalen Ende definiert; und
einen Drucksensor (19), der zur Messung biologischen Drucks ausgelegt ist, wobei mindestens ein Teil des Drucksensors (19) innerhalb des Rohrs (31) montiert ist, und wobei der Drucksensor (19) einen Drucksensorwandler (29) umfasst,
die Umfangswandung (32) des Rohrs (31) genau drei Öffnungen (35, 36, 37) enthält, die für Fluid- und Gaseinlass und -auslass, die sich radial dadurch erstrecken, ausgelegt sind: eine erste längliche Öffnung (35), eine zweite längliche Öffnung (36) und eine dritte längliche Öffnung (37),
wobei jede der ersten, zweiten und dritten länglichen Öffnung (35, 36, 37) in der Axialrichtung des Führungsdrahts (300) länglich ist,
wobei eine Mehrheit jeder der ersten, zweiten und dritten länglichen Öffnung (35, 36, 37) innerhalb einer distalen Hälfte des Rohrs (31) angeordnet ist,
die erste längliche Öffnung (35) an einer dem Drucksensorwandler (29) entgegengesetzten Stelle angeordnet ist, so dass der Drucksensorwandler (29) über die erste längliche Öffnung (35) freigelegt ist, **dadurch gekennzeichnet, dass**
die zweite längliche Öffnung (36) so angeordnet ist, dass in einer Querschnittsansicht ein Bisektor (B₂) der zweiten länglichen Öffnung (36) von einem Bisektor (B₁) der ersten länglichen Öffnung (35) in einer ersten Umfangsrichtung um einen Winkel (θ₁) in einem Bereich von 95 bis 115 Grad versetzt ist, und
die dritte längliche Öffnung (37) so angeordnet ist, dass in der Querschnittsansicht ein Bisektor (B₃) der dritten länglichen Öffnung (37) vom Bisektor (B₁) der ersten länglichen Öffnung (35) in einer zweiten Umfangsrichtung um einen Winkel (θ₂) in einem Bereich von 95 bis 115 Grad versetzt ist,
wobei in der Querschnittsansicht ein Umfangswinkel (ϕ_{O1}), über den sich die erste längliche Öffnung (35) erstreckt, größer ist als ein Umfangswinkel (ϕ_{O2}), über den sich die zweite längliche Öffnung (36) erstreckt, und ein Umfangswinkel (ϕ_{O3}), über den sich die dritte längliche Öffnung (37) erstreckt.

2. Führungsdraht (300) nach Anspruch 1, wobei in der Querschnittsansicht der Umfangswinkel (ϕ_{O1}), über den sich die erste längliche Öffnung (35) erstreckt, in einem Bereich von 65 bis 75 Grad liegt, und der Umfangswinkel (ϕ_{O2}), über den sich die zweite längliche Öffnung (36) erstreckt, und der Umfangswinkel (ϕ_{O3}), über den sich die dritte längliche Öffnung (37) erstreckt, jeweils in einem Bereich von 35 bis 45 Grad liegen.

3. Führungsdraht (300) nach Anspruch 1 oder Anspruch 2, wobei in der Querschnittsansicht eine Länge einer Kordel (C_{O1}), die sich zwischen entgegengesetzten Kanten der ersten länglichen Öffnung (35) erstreckt, in einem Bereich von 0,18 bis 0,22 mm liegt, und eine Länge einer Kordel (C_{O2}), die sich zwischen entgegengesetzten Kanten der zweiten länglichen Öffnung (36) erstreckt, und eine Länge einer Kordel (C_{O3}), die sich zwischen entgegengesetzten Kanten der dritten länglichen Öffnung (37) erstreckt, jeweils in einem Bereich von 0,07 bis 0,17 mm liegen.

4. Führungsdraht (300) nach einem der Ansprüche 1 bis 3, wobei ein Außendurchmesser des Rohrs (31) in einem Bereich von 0,33 bis 0,39 mm liegt, und ein Innendurchmesser des Rohrs (31) in einem Bereich von 0,26 bis 0,32 mm liegt.

5. Führungsdraht (300) nach einem der Ansprüche 1 bis 4, wobei:
Teile des Rohrs (31) eine zwischen der ersten länglichen Öffnung (35) und der zweiten länglichen Öffnung (36) angeordnete erste Strebe (38) und eine zwischen der ersten länglichen Öffnung (35) und der dritten länglichen Öffnung (37) angeordnete zweite Strebe (39) bilden, und
in der Querschnittsansicht der Umfangswinkel (ϕ_{S1}), über den sich die erste Strebe (38) erstreckt, und der Umfangswinkel (ϕ_{S2}), über den sich die zweite Strebe (39) erstreckt, jeweils in einem Bereich von 40 bis 50 Grad liegen.

6. Führungsdraht (300) nach Anspruch 5, wobei in der Querschnittsansicht eine Länge einer Kordel (C_{S1}), die sich zwischen entgegengesetzten Kanten der ersten Strebe (38) erstreckt, und eine Länge einer Kordel (C_{S2}), die sich zwischen entgegengesetzten Kanten der zweiten Strebe (39) erstreckt, jeweils in einem Bereich von 0,08 bis 0,22 mm liegen.

7. Führungsdraht (300) nach einem der Ansprüche 1 bis 6, wobei proximale Enden (35a, 36a, 37a) der ersten, zweiten und dritten länglichen Öffnung (35, 36, 37) in einer Umfangsrichtung ausgerichtet sind, und distale Enden (35b, 36b, 37b) der ersten, zweiten und dritten länglichen Öffnung (35, 36, 37) in einer Umfangsrichtung ausgerichtet sind.

8. Führungsdraht (300) nach Anspruch 7, wobei ein Axialabstand (L_{DD}) zwischen einem distalen Ende (31b) des Rohrs (31) und jedem der distalen Enden (35a, 36a, 37a) der ersten, zweiten und dritten länglichen Öffnung (35, 36, 37) in einem Bereich von 0,18 bis 0,28 mm liegt.

9. Führungsdraht (300) nach Anspruch 8, wobei ein Axialabstand (L_{DP}) zwischen dem distalen Ende (31b) des Rohrs (31) und jedem der proximalen Enden (35a, 36a, 37a) der ersten, zweiten und dritten länglichen Öffnung (35, 36, 37) in einem Bereich von 0,84 bis 0,94 mm liegt.

10. Führungsdraht (300) nach einem der Ansprüche 1 bis 9, wobei eine Axiallänge (Lo) der ersten länglichen Öffnung (35), eine Axiallänge (Lo) der zweiten länglichen Öffnung (36) und eine Axiallänge (Lo) der dritten länglichen Öffnung (37) jeweils in einem Bereich von 27 bis 37 % einer Länge des Rohrs (31) liegen.

11. Führungsdraht (300) nach einem der Ansprüche 1 bis 10, wobei eine Axiallänge (Lo) der ersten länglichen Öffnung (35), eine Axiallänge (Lo) der zweiten länglichen Öffnung (36) und eine Axiallänge (Lo) der dritten länglichen Öffnung (37) jeweils in einem Bereich von 0,55 bis 0,75 mm liegen.

12. Führungsdraht (300) nach einem der Ansprüche 1 bis 11, wobei die erste längliche Öffnung (35) eine Form eines Rechtecks mit abgerundeten Ecken aufweist.

13. Führungsdraht (300) nach Anspruch 12, wobei jede der zweiten und dritten länglichen Öffnung (35, 36, 37) eine Form eines Rechtecks mit kreisförmigen Segmenten an proximalen und distalen Enden davon aufweist.

14. Führungsdraht (300) nach einem der Ansprüche 1 bis 13, wobei:
in der Querschnittsansicht der Bisektor (B₂) der zweiten länglichen Öffnung (36) vom Bisektor (B₁) der ersten länglichen Öffnung (35) in der ersten Umfangsrichtung um einen Winkel (θ₁) in einem Bereich von 95 bis 105 Grad versetzt ist, und
in der Querschnittsansicht der Bisektor (B₃) der dritten länglichen Öffnung (37) vom Bisektor (B₁) der ersten länglichen Öffnung (35) in der zweiten Umfangsrichtung um einen Winkel (θ₂) in einem Bereich von 95 bis 105 Grad versetzt ist.

15. Führungsdraht (300) nach einem der Ansprüche 1 bis 14, wobei eine Gesamtheit jeder der ersten, zweiten und dritten länglichen Öffnung (35, 36, 37) innerhalb der distalen Hälfte des Rohrs (31) angeordnet ist.

## Revendications

1. Fil de guidage (300) pour la mesure de pression biologique, comprenant :
un tube (31) s'étendant le long d'une direction axiale du fil de guidage (300), le tube (31) comprenant une paroi circonférentielle (32) et définissant une ouverture d'extrémité proximale (33) et une ouverture d'extrémité distale (34) ; et
un capteur de pression (19) configuré pour la mesure de pression biologique, au moins une partie du capteur de pression (19) étant montée à l'intérieur du tube (31), et le capteur de pression (19) comprenant un transducteur de capteur de pression (29),
la paroi circonférentielle (32) du tube (31) comprenant exactement trois ouvertures (35, 36, 37) configurées pour l'entrée et la sortie de fluide et de gaz s'étendant radialement à travers celle-ci : une première ouverture allongée (35), une deuxième ouverture allongée (36) et une troisième ouverture allongée (37),
dans lequel chacune des première, deuxième et troisième ouvertures allongées (35, 36, 37) est allongée dans la direction axiale du fil de guidage (300),
dans lequel une majorité de chacune des première, deuxième et troisième ouvertures allongées (35, 36, 37) est située à l'intérieur d'une moitié distale du tube (31),
la première ouverture allongée (35) est située à un emplacement opposé au transducteur de capteur de pression (29) si bien que le transducteur de capteur de pression (29) est exposé via la première ouverture allongée (35),
**caractérisé en ce que**
la deuxième ouverture allongée (36) est située si bien que, dans une vue en coupe transversale, une bissectrice (B₂) de la deuxième ouverture allongée (36) est décalée d'une bissectrice (B₁) de la première ouverture allongée (35) dans une première direction circonférentielle d'un angle (θ₁) dans une plage de 95 à 115 degrés, et
la troisième ouverture allongée (37) est située si bien que, dans la vue en coupe transversale, une bissectrice (B₃) de la troisième ouverture allongée (37) est décalée d'une bissectrice (B₁) de la première ouverture allongée (35) dans une deuxième direction circonférentielle d'un angle (θ₂) dans une plage de 95 à 115 degrés,
dans lequel, dans la vue en coupe transversale, un angle circonférentiel (ϕ_{O1}) sur lequel s'étend la première ouverture allongée (35) est supérieur à un angle circonférentiel (ϕ_{O2}) sur lequel s'étend la deuxième ouverture allongée (36) et un angle circonférentiel (ϕ_{O3}) sur lequel s'étend la troisième ouverture allongée (37).

2. Fil de guidage (300) selon la revendication 1, dans lequel, dans la vue en coupe transversale, l'angle circonférentiel (ϕ_{O1}) sur lequel s'étend la première ouverture allongée (35) dans une plage de 65 à 75 degrés, et l'angle circonférentiel (ϕ_{O2}) sur lequel s'étend la deuxième ouverture allongée (36), et l'angle circonférentiel (ϕ_{O3}) sur lequel s'étend la troisième ouverture allongée (37) sont chacun dans une plage de 35 à 45 degrés.

3. Fil de guidage (300) selon la revendication 1 ou la revendication 2, dans la vue en coupe transversale, une longueur d'une corde (C_{O1}) s'étendant entre les bords opposés de la première ouverture allongée (35) est dans une plage de 0,18 à 0,22 mm, et une longueur d'une corde (C_{O2}) s'étendant entre les bords opposés de la deuxième ouverture allongée (36) et une longueur d'une corde (C_{O3}) s'étendant entre les bords opposés de la troisième ouverture allongée (37) sont chacune dans une plage de 0,07 à 0,17 mm.

4. Fil de guidage (300) selon l'une quelconque des revendications 1 à 3, dans lequel un diamètre extérieur du tube (31) est dans une plage de 0,33 à 0,39 mm, et un diamètre intérieur du tube (31) est dans une plage de 0,26 à 0,32 mm.

5. Fil de guidage (300) selon l'une quelconque des revendications 1 à 4, dans lequel :
des parties du tube (31) forment une première entretoise (38) située entre la première ouverture allongée (35) et la deuxième ouverture allongée (36), et une deuxième entretoise (39) située entre la première ouverture allongée (35) et la troisième ouverture allongée (37), et
dans la vue en coupe, l'angle circonférentiel (ϕ_{S1}) sur lequel s'étend la première entretoise (38) et l'angle circonférentiel (ϕ_{S2}) sur lequel s'étend la deuxième entretoise (39), sont chacun dans une plage de 40 à 50 degrés.

6. Fil de guidage (300) selon la revendication 5, dans lequel, dans la vue en coupe transversale, une longueur d'une corde (C_{S1}) s'étendant entre les bords opposés de la première entretoise (38) et une longueur d'une corde (C_{S2}) s'étendant entre les bords opposés de la deuxième entretoise (39) sont chacune dans une plage de 0,08 à 0,22 mm.

7. Fil de guidage (300) selon l'une quelconque des revendications 1 à 6, dans lequel les extrémités proximales (35a, 36a, 37a) des première, deuxième et troisième ouvertures allongées (35, 36, 37) sont alignées dans une direction circonférentielle, et les extrémités distales (35b, 36b, 37b) des première, deuxième et troisième ouvertures allongées (35, 36, 37) sont alignées dans une direction circonférentielle.

8. Fil de guidage (300) selon la revendication 7, dans lequel une distance axiale (L_{DD}) entre une extrémité distale (31b) du tube (31) et chacune des extrémités distales (35b, 36b, 37b) des première, deuxième et troisième ouvertures allongées (35, 36, 37) est dans une plage de 0,18 à 0,28 mm.

9. Fil de guidage (300) selon la revendication 8, dans lequel une distance axiale (L_{DP}) entre l'extrémité distale (31b) du tube (31) et chacune des extrémités distales (35b, 36a, 37a) des première, deuxième et troisième ouvertures allongées (35, 36, 37) est dans une plage de 0,84 à 0,94 mm.

10. Fil de guidage (300) selon l'une quelconque des revendications 1 à 9, dans lequel une longueur axiale (Lo) de la première ouverture allongée (35), une longueur axiale (Lo) de la deuxième ouverture allongée (36), et une longueur axiale (Lo) de la troisième ouverture allongée (37) sont chacune dans une plage de 27 à 37% d'une longueur du tube (31).

11. Fil de guidage (300) selon l'une quelconque des revendications 1 à 10, dans lequel une longueur axiale (Lo) de la première ouverture allongée (35), une longueur axiale (Lo) de la deuxième ouverture allongée (36), et une longueur axiale (Lo) de la troisième ouverture allongée (37) sont chacune dans une plage de 0,55 à 0,75 mm.

12. Fil de guidage (300) selon l'une quelconque des revendications 1 à 11, dans lequel la première ouverture allongée (35) présente la forme d'un rectangle avec des coins arrondis.

13. Fil de guidage (300) selon la revendication 12, dans lequel chacune des deuxième et troisième ouvertures allongées (35, 36, 37) présente la forme d'un rectangle avec des segments circulaires à ses extrémités proximale et distale.

14. Fil de guidage (300) selon l'une quelconque des revendications 1 à 13, dans lequel :
dans la vue en coupe transversale, la bissectrice (B₂) de la deuxième ouverture allongée (36) est décalée de la bissectrice (B₁) de la première ouverture allongée (35) dans la première direction circonférentielle d'un angle (θ₁) dans une plage de 95 à 105 degrés, et
dans la vue en coupe transversale, la bissectrice (B₃) de la troisième ouverture allongée (37) est décalée de la bissectrice (B₁) de la première ouverture allongée (35) dans la deuxième direction circonférentielle d'un angle (θ₂) dans une plage de 95 à 105 degrés.

15. Fil de guidage (300) selon l'une quelconque des revendications 1 à 14, dans lequel une totalité de chacune des première, deuxième et troisième ouvertures allongées (35, 36, 37) est située à l'intérieur de la moitié distale du tube (31).
